# EUROPEAN PATENT APPLICATION

(11) **EP 4 834 239 A1**
(43) Date of publication of application: **04.11.2026**
(21) Application number: 23962707.8
(22) Date of filing: 28.12.2023
(51) Int. Cl.: A61B 10/00

(54) **POLYP RETRIEVAL DEVICE, FILTER THEREOF, AND SUPPORT THEREOF**

(71) Applicant: Chau, King Yuen, NT, Hong Kong (HK)
(72) Inventor: Chau, King Yuen, NT, Hong Kong (HK)
(74) Representative: Isern Patentes y Marcas S.L.
(86) International application number: PCT/CN2023/142914
(87) International publication number: WO 2025/138028

(57) **Abstract**

The present application discloses a polyp recovery apparatus and a filter and stent thereof, wherein the filter applied to the polyp recovery apparatus comprises a body, and a liquid injection hole and a liquid filtration mechanism that are disposed on the body, the body comprises an upper shell and a lower shell that are buckled to each other, and an accommodating cavity for recovering polyps is formed between the upper shell and the lower shell. According to the polyp recovery apparatus and the filter and stent thereof disclosed in the present application, since the body of the filter adopts a structure in which the upper shell and the lower shell are hinged, when the filter is inserted into the stent, the upper shell and the lower shell are buckled to each other to form the accommodating cavity, so that the polyps can be prevented from splashing out of the filter, thereby preventing living tissue sample loss caused by discharge of the sample from the stent, ensuring that there are enough samples for a doctor to complete pathological analysis, reducing the probability of a second biopsy, preventing a patient from unnecessary pain, and saving medical resources. Meanwhile, there is no need to manually collect polyp samples remaining on an inner wall of the stent, thereby reducing the difficulty of cleaning the stent.

## Description

### Technical Field

The present invention relates to the technical field of medical instruments, in particular to a polyp recovery apparatus and a filter and stent thereof.

### Background Art

Polyps are abnormal proliferative tissues commonly found in the colon, stomach, throat, nasal cavity, ear canals, bladder, and uterus. Doctors need to obtain living tissue samples, perform pathological analysis on the samples, and determine whether the polyps are malignant or benign in nature to accordingly determine whether the polyps need to be resected from patients. Obtaining the living tissue samples belongs to an invasive surgery. A small number of polyps need to be resected from the bodies of the patients using invasive tools to serve as the living tissue samples. In a surgical process, a certain dose of medicinal liquid needs to be injected into lesion locations first to achieve the effects of marking colors and controlling vasoconstriction at polyp sites, and then the polyps are resected and aspirated into a polyp recovery apparatus through surgical instruments. In this process, part of blood and tissue fluid as well as the medicinal liquid are mixed with the polyps to form aspirated liquid. Therefore, the aspirated liquid aspirated into the polyp recovery apparatus is mixed liquid of multiple substances. The liquid in a container needs to be filtered through a polyp recovery device to filter out the liquid in the aspirated liquid to obtain qualified living tissue samples that can be used for pathological analysis.

An existing polyp recovery device comprises a stent and a filter. The stent has a liquid filtration cavity for fixing the filter. When the polyps are recovered, the polyps are collected inside the filter through filtration of the filter, and the filtered liquid is continually drained out of the stent.

However, when the existing polyp recovery device is used, the polyps are rushed out of the filter and adhered to an inner wall of the stent, so that some living tissue samples are lost. Insufficient samples cause doctors to be unable to complete pathological analysis, or affect the accuracy of pathological analysis results. Therefore, patients are often required to undergo a second biopsy to re-obtain tissue samples, which increases the pain of patients and wastes medical resources.

### Summary

Embodiments of the present invention aim to provide a polyp recovery apparatus and a filter and stent thereof, which can solve the technical problem that polyp samples are easily lost in a polyp recovery apparatus in the prior art.

The embodiments of the present invention adopt the following technical solutions to solve the technical problem:

According to a first aspect of the present application, the present application discloses a filter applied to a polyp recovery apparatus, comprising:
a body, wherein the body comprises an upper shell and a lower shell that are buckled to each other, and an accommodating cavity for recovering polyps is formed between the upper shell and the lower shell;
a liquid injection hole formed in the upper shell and used for aspirated liquid to enter the accommodating cavity;
a liquid filtration mechanism disposed on the lower shell and used for filtering out moisture inside the accommodating cavity to filter the polyps.

According to the filter applied to a polyp recovery apparatus disclosed in the embodiments of the present application, since the body of the filter adopts a structure in which the upper shell and the lower shell are hinged, when the filter is inserted into the stent, the upper shell and the lower shell are buckled to each other to form the accommodating cavity, so that the polyps can be prevented from splashing out of the filter, thereby preventing living tissue sample loss caused by discharge of the sample from the stent, ensuring that there are enough samples for a doctor to complete pathological analysis, reducing the probability of a second biopsy, preventing a patient from unnecessary pain, and saving medical resources. Meanwhile, in the present application, the polyps are prevented from adhering to an inner wall of the stent, so that there is no need to manually collect polyp samples remaining on the inner wall of the stent, and the difficulty of cleaning the stent can be reduced, making the apparatus more convenient to use.

According to a second aspect of the present application, the present application further discloses a stent applied to a polyp recovery device, comprising:
a liquid filtration cavity used for accommodating a filter;
an assembly port disposed on a surface of the stent for allowing the filter to be inserted therein and being in communication with the liquid filtration cavity;
a flow channel joint comprising a liquid injection joint and a liquid drainage joint, wherein the liquid injection joint is used for being in communication with the liquid injection hole of the filter, and the liquid drainage joint is in communication with the liquid filtration cavity for draining liquid inside the liquid filtration cavity.

The filter is accommodated through the liquid filtration cavity of the stent; the assembly port allows the filter to be inserted in or pulled out; the liquid injection joint of the stent is in communication with the liquid injection hole of the filter to prevent polyps from splashing from the accommodating cavity of the filter to an inner wall of the stent, thus having no need to additionally clean the liquid filtration cavity of the stent, and making the entire apparatus faster and more convenient to use.

According to a third aspect of the present application, the present application further discloses a polyp recovery apparatus, comprising the above-mentioned filter and a stent for fixing the filter; when it needs to recover polyps in aspirated liquid, the filter is inserted into the stent, the polyps in the aspirated liquid are collected inside the accommodating cavity through the filter, and liquid filtered out by the filter is drained from a liquid drainage joint of the stent. The filter is then pulled out of the stent, and the polyps inside the filter are transferred to a specific container for storage.

### BRIEF DESCRIPTION OF THE DRAWINGS

One or more embodiments are exemplarily illustrated through figures in drawings corresponding thereto. These exemplary illustrations do not constitute limitations on the embodiments. Elements with the same reference numerals in the drawings represent similar elements. Unless otherwise stated, the figures in the drawings do not constitute limitations on the scale.
FIG. 1 is a structural schematic diagram of a stent in the embodiments of the present application;
FIG. 2 is a structural schematic diagram of a filter in the embodiments of the present application;
FIG. 3 is an assembly diagram of the filter in the embodiments of the present application;
FIG. 4 is a structural schematic diagram of a body in an open state in the embodiments of the present application;
FIG. 5 is a structural schematic diagram of a handle in the embodiments of the present application;
FIG. 6 is an assembly diagram of the stent in the embodiments of the present application;
FIG. 7 is a structural schematic diagram of the filter in a closed state in yet another embodiment of the present application;
FIG. 8 is a structural schematic diagram of an adjustment device in yet another embodiment of the present application;
FIG. 9 is a structural schematic diagram of an adjustment device in yet another embodiment of the present application.
FIG. 10 is an assembly diagram of the stent in yet another embodiment of the present application.

### Drawing symbols and corresponding meanings:

filter 1, body 10, accommodating cavity 100, guide column 101, rotating shaft 102, baffle 103, sealing ring 104, clamping slot 105, groove 106, air hole 107, upper shell 11, upper mounting portion 110, pin shaft 111, expansion buckle 1110, first fixing groove 112, lower shell 12, lower mounting portion 120, shaft sleeve 121, second fixing groove 122, liquid injection hole 13, sinking groove 130, liquid filtration hole 14, torsion spring 15, first support leg 151, second support leg 152, sealing device 16, piston 160, sealing plug 161, handle 17, first contact face 171, second contact face 172, extension portion 173, compression spring 18;
stent 2, liquid filtration cavity 20, assembly port 21, liquid injection joint 22, liquid drainage joint 23, sealing interface 24, sealing portion 241, guide portion 242, positioning protrusion 25, top plate 26, bottom plate 27.

### DETAILED DESCRIPTION

In order to facilitate the understanding of the present invention, the present invention is further described in detail below with reference to the accompanying drawings and specific embodiments. It should be noted that when an element is described as being "fixed to" another element, it can be directly on the other element, or one or more intervening elements may be present therebetween. When one element is described as being "connected" to another element, it can be directly connected to the other element, or one or more intervening elements may be present therebetween. The terms "upper", "lower", "inner", "outer", "perpendicular", "horizontal", etc. used in the present specification indicate the orientation or position relationship based on the orientation or position relationship shown in the accompanying drawings, which is only for the convenience of describing the present invention and simplifying the description, rather than indicating or implying that the device or element referred to must have a specific orientation or be constructed and operated in a specific orientation, and therefore cannot be understood as a limitation on the present invention. In addition, the terms "first", "second", etc., are used for descriptive purposes only and cannot be understood as indicating or implying relative importance. "Comprise" or "comprising" and similar words mean that elements or objects appearing before the words include elements or objects listed after the words and their equivalents, without excluding other elements or objects. "Connection" or "connect" and similar words are not limited to physical or mechanical connections, but may include electrical connections, whether direct or indirect. Unless otherwise defined, features such as "parallel", "perpendicular", and "identical" used in the embodiments of the present invention include cases such as "parallel", "perpendicular", and "identical" in a strict sense, as well as cases such as "approximately parallel", "approximately perpendicular", and "approximately identical" containing certain errors. For example, the above "approximately" may represent that a difference between compared objects is within 10% or within 5% of an average value of the compared objects. When a quantity of a component or element is not specified in the following embodiments of the present invention, it means that the component or element can be one or more, or it can be understood as at least one. "At least one" means one or more, and "plurality" means at least two.

Unless otherwise defined, all technical and scientific terms used in the specification have the same meanings as commonly understood by those skilled in the technical field of the present invention. The terms used in the specification of the present invention are only for the purpose of describing specific embodiments and are not used for limiting the present invention. The term "and/or" used in the specification includes any and all combinations of one or more of associated listed items.

In addition, technical features involved in different embodiments of the present invention described below may be combined with each other as long as there is no conflict between them. Obviously, the described embodiments are some, but not all, of the embodiments of the present invention. Based on the embodiments of the present invention described, all other embodiments obtained by those of ordinary skill in the art without exertion of any inventive work fall within the scope of protection of the present invention.

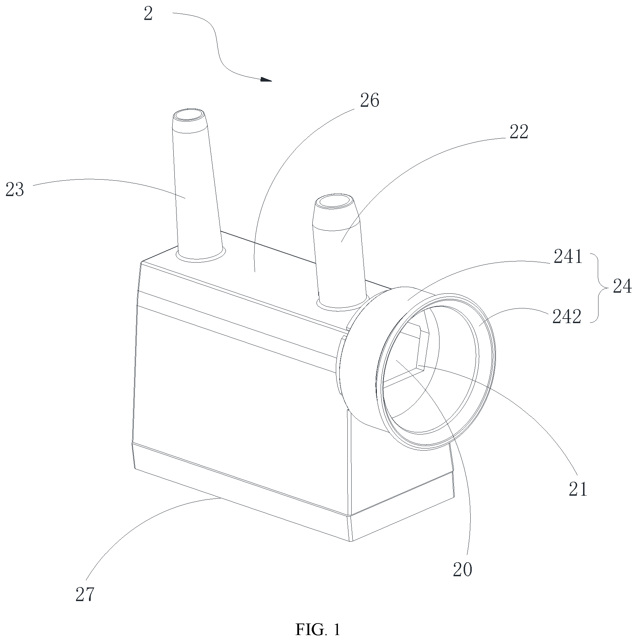

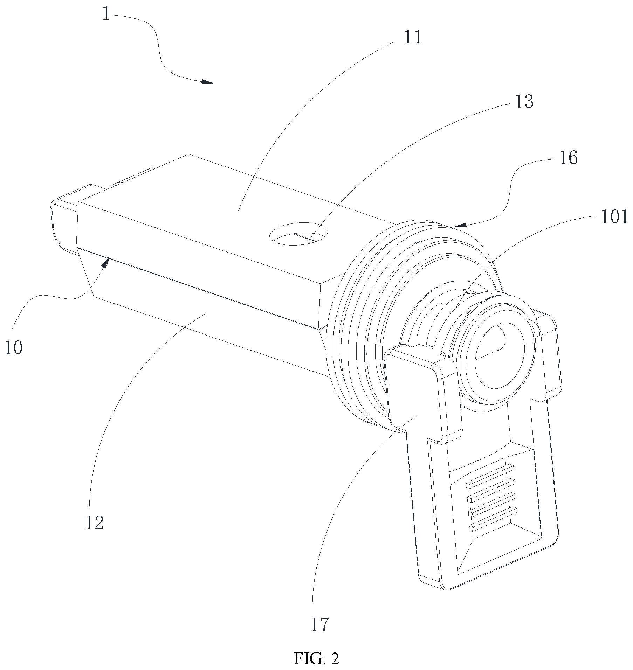

A polyp recovery apparatus disclosed in the present application, as shown in FIG. 1 and FIG. 2, comprises a filter 1 for filtering polyps from aspirated liquid, and a stent 2 for fixing the filter 1. The stent 2 is of a hollow structure, with a liquid filtration cavity 20 for accommodating the filter 1 formed inside the stent; a side face of the stent 2 is provided with an assembly port 21 for allowing the filter 1 to be inserted in and pulled out; the stent 2 is further provided with a liquid injection joint 22 for injecting the aspirated liquid into the filter 1, and a liquid drainage joint 23 for draining the liquid filtered out by the filter 1. The filter 1 comprises a body 10, the body 10 comprises an upper shell 11 and a lower shell 12 that are buckled to each other, and the upper shell 11 and the lower shell 12 are buckled to form an accommodating cavity 100 for recovering the polyps. The upper shell 11 is provided with a liquid injection hole 13, and the lower shell 12 is provided with a liquid filtration mechanism, wherein the liquid injection hole 13 is used for docking with the liquid injection joint 22; the aspirated liquid mixed with the polyps is injected into the accommodating cavity 100 through the liquid injection hole 13, moisture in the aspirated liquid is filtered out of the body 10 through the liquid filtration mechanism, the filtered moisture enters the liquid filtration cavity 20, the stent 2 is finally pulled out through a negative pressure pump at another end of the liquid drainage joint 23, and then a polyp sample inside the filter 1 is transferred to a living tissue sample recovery vessel (a designated vessel soaked in medicinal liquid), thus achieving recovery and preservation of the polyps. In this embodiment, the liquid filtration mechanism is a plurality of liquid filtration holes 14 disposed on a bottom face of the lower shell 12; in other embodiments of the present application, the liquid filtration cavity 20 may also be a filter screen fixed to the lower shell 12.

As disclosed in this embodiment, since the body 10 of the filter 1 adopts a structure in which the upper shell 11 and the lower shell 12 are buckled, when the filter 1 is inserted into the stent 2, the upper shell 11 and the lower shell 12 are buckled to each other to form the accommodating cavity 100, so that the polyps can be prevented from splashing out of the filter 1, thereby preventing an increase in the difficulty of cleaning the stent 2 caused by the polyps adhering to an inner wall of the stent 2, and making the apparatus more convenient to use.

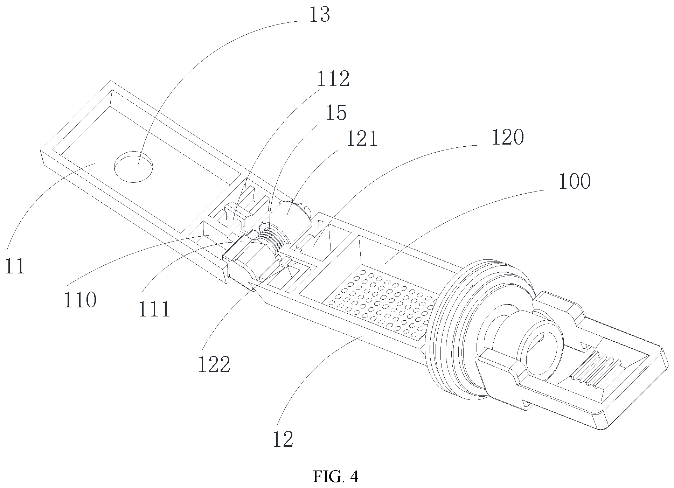

The body 10 has a first end and a second end along a length direction, and the upper shell 11 and the lower shell 12 are hinged at the first end of the body 10, thus enabling the upper shell 11 and the lower shell 12 to be closed or opened through a hinged connection. When the polyps are filtered, as shown in FIG. 2, the upper shell 11 and the lower shell 12 stay in a buckled state, thus preventing sample loss caused by the polyps splashing to an inner wall of the liquid filtration cavity 20; when the recovered polyp sample needs to be stored in a designated living tissue sample storage vessel, only the filter 1 needs to be pulled out from the stent 2, and at this time, as shown in FIG. 4, the upper shell 11 and the lower shell 12 stay in an open state, thus facilitating the transfer of the polyps inside the lower shell 12 to the designated vessel.

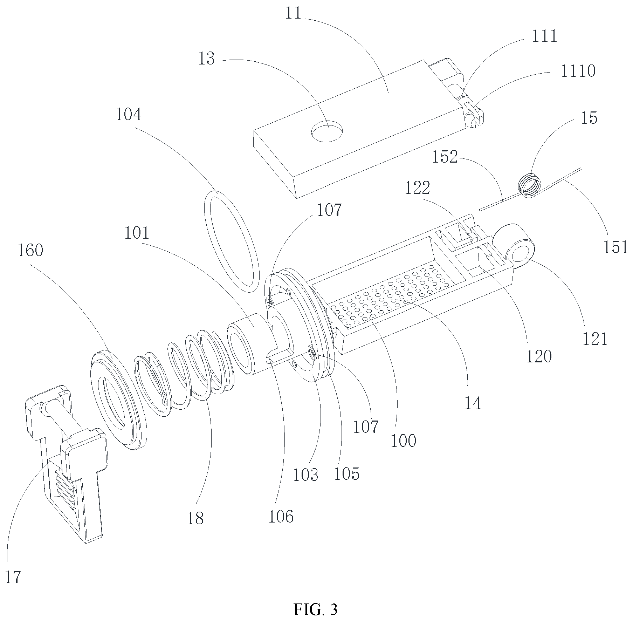

Specifically, as shown in FIG. 3 and FIG. 4, the upper shell 11 and the lower shell 12 are respectively provided with an upper mounting portion 110 and a lower mounting portion 120 at the first end of the body 10, and the upper mounting portion 110 is disposed opposite to the lower mounting portion 120. The upper assembling portion 110 and the lower assembling portion 120 are disposed on an outer side of the accommodating cavity 100, thus enabling the accommodating cavity 100 to be independently disposed, and maintaining the accommodating cavity 100 with sealing performance when the upper shell 11 and the lower shell 12 are buckled. The first mounting portion 110 is fixed with a pin shaft 111, the lower mounting portion 120 is provided with a shaft sleeve 121, and the pin shaft 111 is fitted with the shaft sleeve 121, thus achieving a hinged connection between the upper shell 11 and the lower shell 12 through the pin shaft 111. A root portion of the pin shaft 111 is fixedly connected to the first mounting portion 110, an end portion of the pin shaft passes through the shaft sleeve 121, the end portion of the pin shaft 111 is provided with an expansion buckle 1110 fitted with the shaft sleeve 121, and the expansion buckle 1110 is connected to an edge of the shaft sleeve 121 in a clamped mode, thus preventing the upper shell 11 and the lower shell 12 from being separated from each other along an axial direction of the pin shaft 111.

In order to facilitate operation, the first end of the body 10 is provided with a first reset member for allowing the upper shell 11 and the lower shell 12 to rotate toward an open direction. In this embodiment, the first reset member is a torsion spring 15 disposed at a hinge joint between the upper shell 11 and the lower shell 12; the torsion spring 15 is sleeved on the pin shaft 111; the torsion spring 15 comprises a first support leg 151 and a second support leg 152; the upper mounting portion 110 is provided with a first fixing groove 112 for fixing the first support leg 151, and the first support leg 151 is inserted into the first positioning groove 112 to abut against an inner surface of the upper shell 11; the lower mounting portion 120 is provided with a second fixing groove 122 for fixing the second support leg 152, and the second support leg 152 is inserted into the second fixing groove 122 to abut against an inner surface of the lower shell 12.

In this way, after the body 10 recovers the polyps, as long as a restraining force exerted on the body 10 is removed, the upper shell 11 and the lower shell 12 are automatically opened under an action of the torsion spring 15, thereby facilitating the transfer of the polyp sample inside the accommodating cavity 100 to a specific living tissue sample recovery vessel, and also facilitating the cleaning of the filter 1. In addition, if the first support leg 151 and the second support leg 152 extend into the accommodating cavity 100, additional avoidance structures need to be provided on inner walls of the upper shell 11 and the lower shell 12 to ensure that the torsion spring 15 does not destroy a sealing structure of the accommodating cavity 100. In this embodiment, the upper mounting portion 110 and the lower mounting portion 120 are disposed outside the accommodating cavity 100, which not only does not destroy the sealing performance of the accommodating cavity 100 when the body 10 is buckled, but also reduces the difficulty of machining the upper shell 11 and the lower shell 12, thereby reducing a production cost of a product.

As shown in FIG. 1 and FIG. 2, the assembly port 21 is fitted with a cross-sectional shape of the body 10 in a buckled state, so that when the filter 1 is inserted into the assembly port 21, the upper shell 11 and the lower shell 12 are automatically buckled under an action of the assembly port 21. In this embodiment, the assembly port 21 is in a polygon shape, and correspondingly, a cross section of the body 10 in the buckled state is in a polygon shape that matches the assembly port 21. In this way, the assembly port 21 can not only allow the body 10 to be automatically buckled when inserted, but also prevent the body 10 from rotating inside the assembly port 21, thereby preventing the liquid injection hole 13 from being misaligned with the liquid injection joint 22.

Since the assembly port 21 matches the cross section of the body 10, the first end of the body 10 with the upper shell 11 and the lower shell 12 in the open state is inserted into the assembly port 21, and with the insertion of the first end of the body 10, the upper shell 11 and the lower shell 12 are automatically buckled under an action of the assembly port 21. On the contrary, when the body 10 is pulled out of the assembly port 21, the upper shell 11 and the lower shell 12 are automatically opened under an action of the torsion spring 15 due to the loss of constraint of the assembly port 21. In this way, there is no need to actively buckle the upper shell 11 and the lower shell 12 when the body 10 is inserted in or pulled out, and one-handed operation can be achieved, which brings great convenience to use.

When the polyp recovery apparatus is in operation, in order to prevent the aspirated liquid from flowing out of the liquid filtration cavity 20, the sealing performance of the assembly port 21 needs to be maintained. In order to achieve this function, a sealing device 16 is further provided at the second end of the body 10, and a sealing effect is achieved by fitting the sealing device 16 with the assembly port 21.

In this embodiment, as shown in FIG. 3, the sealing device 16 is a piston 160 disposed at the second end of the body 10, and the piston 160 is fitted with the assembly port 21 and plays a sealing role when the body 10 is inserted into the assembly port 21. Specifically, the second end of the body 10 is provided with a guide column 101, a root portion of the guide column 101 is fixedly connected to an outer wall of the lower shell 12, and an end portion of the guide column extends along a direction away from the first end of the body 10. The piston 160 is sleeved on the guide column 101 and is slidable along the guide column 101; the filter 1 further comprises an adjustment device; the adjustment device adjusts a position of the piston 160 on the guide column 101 by controlling a sliding direction of the piston, thereby controlling the piston 160 to get in contact with or be detached from the stent 2.

When the polyps need to be recovered from the aspirated liquid, the body 10 is pushed into the stent 2 along a direction of the first end opposite to the assembly port 21; when the liquid injection hole 13 is opposite to the liquid injection joint 22, the adjustment device is controlled to push the piston 160 toward a direction of the assembly port 21, so that the piston 160 is in contact with the stent 2 to achieve sealing of the assembly port 21.

As shown in FIG. 3 to FIG. 5, the adjustment device comprises a handle 17 disposed at one end of the guide column 101 away from the body 10, the handle 17 is movably connected to the guide column 101, and the piston 160 is disposed between the handle 17 and the lower shell 12; a sliding direction of the piston 160 is controlled by operating the handle 17, thereby adjusting a relative position of the piston 160 relative to the assembly port 21.

In this embodiment, the handle 17 is rotationally connected to the guide column 101, and one end of the handle 17 opposite to the piston 160 abuts against the piston 160; by rotating the handle 17, a portion abutting against the piston 160 is changed, thereby controlling the sliding direction of the piston 160. The handle 17 has a first station and a second station on a rotation track; when the handle is located at the first station, as shown in FIG. 4, a distance between the piston 160 and the assembly port 21 is the farthest; when the handle 17 is located at the second station, as shown in FIG. 2 and FIG. 3, a distance between the piston 160 and the assembly port 21 is the closest.

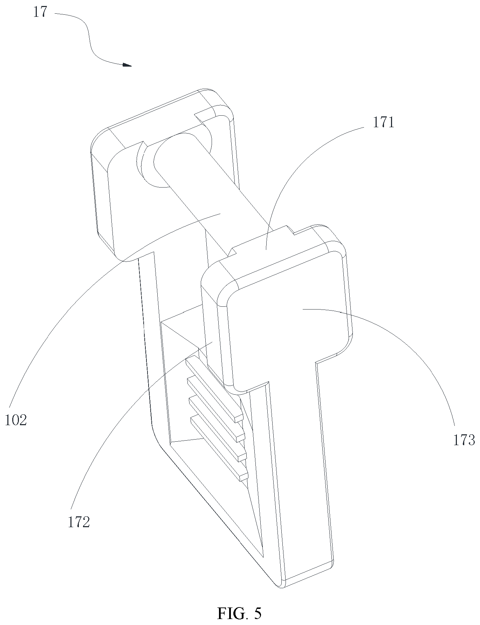

Specifically, as shown in FIG. 3 and FIG. 5, the guide column 101 is provided with the handle 17 that is rotationally connected to the guide column 101 through a rotating shaft 102; the handle 17 is provided with a first contact face 171 and a second contact face 172 at one end opposite to the piston 160; there is an included angle between the first contact face 171 and the second contact face 172; a distance between the rotating shaft 102 and the first contact face 171 is smaller than that between the rotating shaft and the second contact face 172; when the handle 17 is located at the first station, the first contact face 171 abuts against an end face of the piston 160; when the handle 17 is located at the second station, the second contact face 172 abuts against the end face of the piston 160. Since the distance between the rotating shaft 102 and the first contact face 171 is smaller than that between the rotating shaft and the second contact face 172, when the handle 171 rotates from the first station to the second station, the piston 160 is pushed toward the assembly port 21 by the handle 17.

The adjustment device further comprises a second reset member for providing a restoring force required for the piston 160 to slide toward a direction of the handle 17; when the handle 171 rotates from the second station to the first station, the piston 160 is pushed toward the handle 17 by the second reset member, that is, the second reset member pushes the piston 160 toward a direction away from the assembly port. In this embodiment, the second reset member is a compression spring 18 disposed between the lower shell 12 and the piston 160; when the handle 171 rotates from the first station to the second station, the first contact face 171 abutting against the piston 160 is switched to the second contact face 172 abutting against the piston 160, at this time, the piston 160 is pushed toward the assembly port 21 by the handle 17, and the compression spring 18 is in an energy storage state; when the handle 171 rotates from the second station to the first station, the second contact face 172 abutting against the piston 160 is switched to the first contact face 171 abutting against the piston 160, the piston 160 is pushed toward the handle 17 by the compression spring 18, and at this time, the compression spring 18 releases elastic potential energy.

In addition, as shown in FIG. 3 and FIG. 5, a shape of the second contact face 172 of the handle 17 matches the end face of the piston 160; in this embodiment, the second contact face 172 is a flat face, and the corresponding end face of the piston 160 is also a flat face. A positional relationship between the rotating shaft 102 and the second contact face 172 is set such that when the handle 17 rotates to the second station, the second contact face 172 of the handle 17 fits the end face of the piston 160, and a projection of the rotating shaft 102 in a direction perpendicular to the second contact face 172 falls on the second contact face 172. With such a structure, when the handle 17 is located at the second station, pressure exerted by the piston 160 on the second contact face 172 passes through the rotating shaft 102 and is unable to form torque, so that the handle 17 cannot rotate by itself under an action of the compression spring 18, and only by actively rotating the handle 17, the second station can be switched to the first station, thereby constructing a self-locking structure. In this embodiment, the first contact face 171 and the second contact face 172 are perpendicular to each other, so that the handle 17 needs to be rotated 90 degrees to be switched from the first station to the second station.

In order to prevent misoperation when the filter 1 is inserted into the stent 2, the handle 17 is rotated to the second station; in this embodiment, as shown in FIG. 2 and FIG. 5, the first contact face 171 is also set as a flat face that matches the end face of the piston 160, and a positional relationship between the first contact face 171 and the rotating shaft 102 also satisfies that when the handle 17 rotates to the first station, the first contact face 171 of the handle 17 fits the end face of the piston 160, and a projection of the rotating shaft 102 in a direction perpendicular to the first contact face 171 falls on the first contact face 171. In this way, when the handle 17 is located at the first station, pressure exerted by the piston 160 on the first contact face 171 also passes through the rotating shaft 102 and is unable to form torque, so that the handle 17 is unable to rotate from the first station to the second station under slight contact, and only by actively rotating the handle 17 to overcome an elastic force of the compression spring 18, the first station can be switched to the second station. In this way, when the filter 1 is pushed into the stent 2 through the handle 17, the handle 17 is not easily rotated, thereby playing a role in preventing accidental contact and facilitating the completion of an insertion action.

In order to ensure the sealing performance of the assembly port 21, in this embodiment, as shown in FIG. 1, the stent 2 further comprises a sealing interface 24 fixed to the assembly port 21, and the sealing interface 24 is fitted with the sealing device to achieve a sealed connection between the filter and the assembly port when the filter 1 is inserted into the assembly port 21. The sealing interface 24 comprises a sealing portion 241 and a guide portion 242 that are integrally formed, the sealing portion 241 is located at one end opposite to the assembly port 21 and connected to a surface of the stent 2 in a sealed mode, and the guide portion 242 is located at one end away from the assembly port 21. The sealing portion 241 is in a cylindrical shape, and its cross-sectional shape matches the piston 160; when the piston 160 is pushed into the sealing portion 241 along the guide column 101, the piston 160 closely fits an inner wall of the sealing portion 241 to achieve a sealing effect. In this embodiment, in order to facilitate machining, cross sections of the piston 160 and the sealing portion 241 are both in a circular shape. The guide portion 242 is in a funnel shape, and its edge extends circumferentially to form a wide opening with a gradually increasing interface. Since a diameter of the piston 160 is slightly greater than an inner diameter of the sealing portion 241, an interference fit between the two after the piston 160 is pushed into the sealing portion 241 can be ensured to ensure sealing performance. However, this requires a large thrust force for the piston 160 to directly enter the sealing portion 241, which increases an operation difficulty. The guide portion 242 provides the wide opening with an inner diameter greater than that of the piston 160, so that the piston 160 can slide into the sealing portion 241 along a slope of the guide portion 242, and the filter 1 is inserted into the stent 2 more conveniently.

As shown in FIG. 3, one end of the guide column 102 adjacent to the lower shell 11 is further provided with a baffle 103, one end of the baffle 103 opposite to the handle 17 is provided with a groove for fixing the compression spring 18, and an end face of the baffle 103 located at this end is in an annular shape with an outer diameter smaller than that of the piston 160. A side face of the baffle 103 is further provided with a clamping slot 105 for fixing a sealing ring 104, the sealing ring 104 is sleeved inside the clamping slot 105, and the sealing performance of the assembly port 21 is increased by allowing the sealing ring 104 to get in contact with the inner wall of the sealing portion 241. When the handle 17 is located at the second station, the piston 160 is pressed against a surface of the baffle 103 by the handle 17 to play a role in supporting the piston 160, thereby preventing an edge of the piston 160 from curling and affecting a sealing effect. Meanwhile, the compression spring 18 abuts against the baffle 103, thereby preventing the compression spring 18 from directly acting on the lower shell 12 and causing damage to the lower shell 12.

Since the liquid drainage joint 23 of the stent 2 is connected to a negative pressure pump, while the liquid inside the liquid filtration cavity 20 is continuously aspirated by the negative pressure pump, air pressure inside the liquid filtration cavity 20 is lower than the atmospheric pressure. Therefore, affected by a pressure difference between an inside and an outside of the liquid filtration cavity 20, it is difficult to overcome an air pressure resistance and pull out the filter 1. In order to solve the technical problem, in this embodiment, the end face of the baffle 103 is further provided with an air hole 107; when the piston 160 fits the surface of the baffle 103, the piston 160 can cover the air hole 107 to maintain sealing performance of the stent 2. When the filter 1 needs to be pulled out, the piston 160 is detached from the baffle 103 under an action of the compression spring 18, so that the liquid filtration cavity 20 is in communication with the atmosphere outside the stent 2 through a gap between the assembly port 21 and the body 1 as well as the air hole 107, the air pressure inside and outside the liquid filtration cavity 20 is balanced, and convenience is provided for pulling the filter 1 out of the stent 2.

In this embodiment, as shown in FIG. 5, one end of the handle 17 opposite to the guide column 101 is provided with mutually parallel extension portions 173, and the rotating shaft 102 is disposed between the two extension portions 173 and fixedly connected to the extension portions 173. One end of the guide column 101 away from the lower shell 1 is provided with a groove 106 for fixing the rotating shaft 102, an opening 107 of the groove 106 is opposite to the baffle 103, and the groove 106 extends from the opening 107 along an axial direction of the guide column 101 away from the lower shell 12. In this way, when the rotating shaft 102 is placed inside the groove 106, under an action of the compression spring 18, the piston 160 automatically pushes the rotating shaft 102 to one end of the groove 106 away from the lower shell 12, so that the handle 17 cannot fall off by itself. Meanwhile, product assembly is facilitated, and production efficiency is improved.

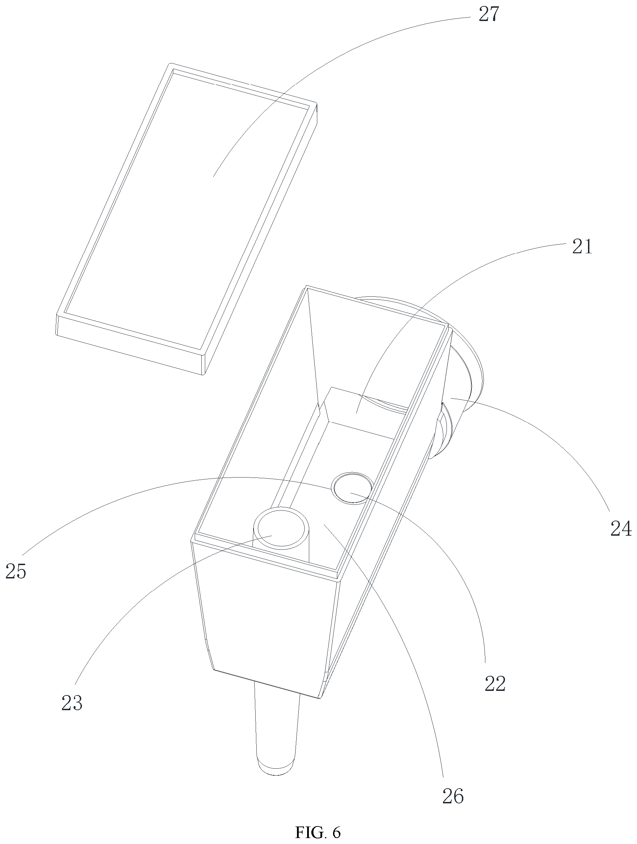

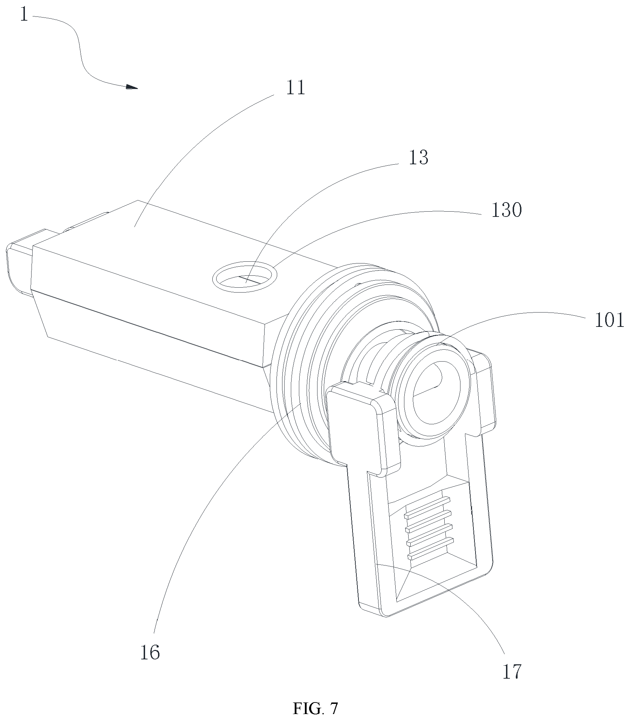

In other embodiments of the present application, as shown in FIG. 6, a positioning protrusion 25 is provided at a connection between the liquid injection joint 22 and the inner wall of the stent 2, the positioning protrusion 25 protrudes from the inner wall of the stent 2, and the positioning protrusion 25 is fitted with the liquid injection hole 13 of the filter 1, so that when the filter 1 enters the liquid filtration cavity 20, the positioning protrusion 25 can slide into the liquid injection hole 13. The positioning protrusion 25 is in an annular shape around an outlet of the liquid injection joint 22; as shown in FIG. 7, a sinking groove 130 fitted with the positioning protrusion 25 is formed at a top portion of the liquid injection hole 13.

In this way, when the filter 1 is pushed into the stent 2, a combination of the positioning protrusion 25 and the liquid injection hole 13 plays a positioning role, ensuring that the liquid injection hole 13 docks with the liquid injection joint 22. Meanwhile, when the piston 160 slides along the guide column 101, cooperation between the positioning protrusion 25 and the liquid injection hole 13 plays a role in fixing the body 10, thereby preventing the liquid injection hole 13 from being misaligned with the liquid injection joint 22 caused by the body 10 moving under an action of the handle 17 or the compression spring 18.

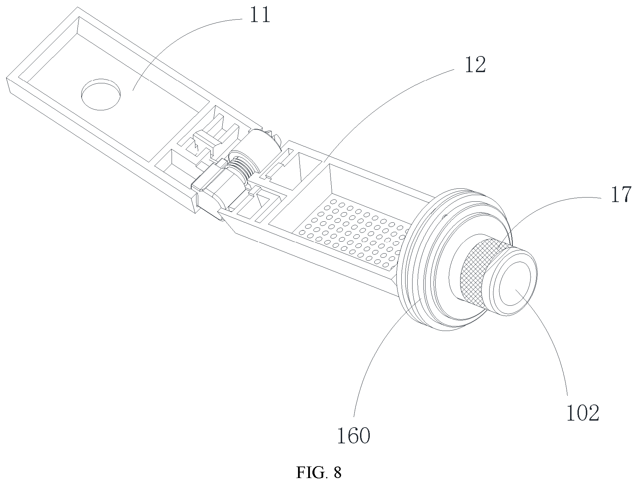

In another embodiment of the present application, as shown in FIG. 8, the handle 17 can also be slidingly connected to the guide column 101, and a moving direction of the piston 160 is controlled by sliding the handle 17. When the filter 1 needs to be installed into the stent 2 and the handle 17 slides toward a direction of the assembly port 21, the piston 160 is pushed to move toward the assembly port 21 until the piston 160 is pushed into the sealing portion 241, and during this process, the compression spring 18 accumulates energy. When the polyps are completely recovered and the filter 1 needs to be pulled out to collect the polyps therein, the handle 17 is pulled back toward a direction away from the lower shell 12, the compression spring 18 pushes the piston 160 to move synchronously with the handle 17, and during this process, the compression spring 18 releases elastic potential energy. The handle 17 is further provided with a buckle structure; when the handle 17 pushes the piston 160 into the sealing portion 241, the buckle structure can lock the handle 17, thereby preventing the compression spring 18 from pushing the piston 160 out of the sealing portion 241.

In another embodiment of the present application, as shown in FIG. 8, the handle 17 can also be fixedly connected to the piston 160 while being slidingly connected to the guide column 101, and the piston 160 is driven to move by sliding the handle 17. In this way, there is no need to dispose the compression spring 18 between the piston 160 and the lower shell 12, and instead, an operator actively pulls the handle 17 back along a direction away from the lower shell 12, thereby dragging the piston 160 out of the sealing portion 241.

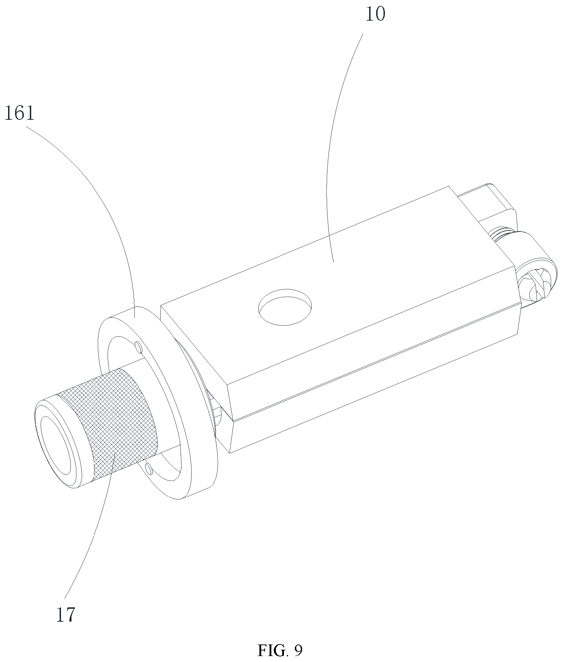

In another embodiment of the present application, as shown in FIG. 9, the sealing device 16 comprises a sealing plug 161 fixed at the second end of the body 10; the sealing plug 161 is fixedly connected to the lower shell 12; when the filter 1 is pushed into the stent 2 and the liquid injection hole 13 docks with the liquid injection joint 22, the sealing plug 161 synchronously enters the sealing portion 241 to achieve sealing of the assembly port 21. In this embodiment, the action of inserting and pulling the filter 1 into and out of the stent 2 is synchronously performed with the action of moving the sealing plug 161 in and out of the sealing portion 241.

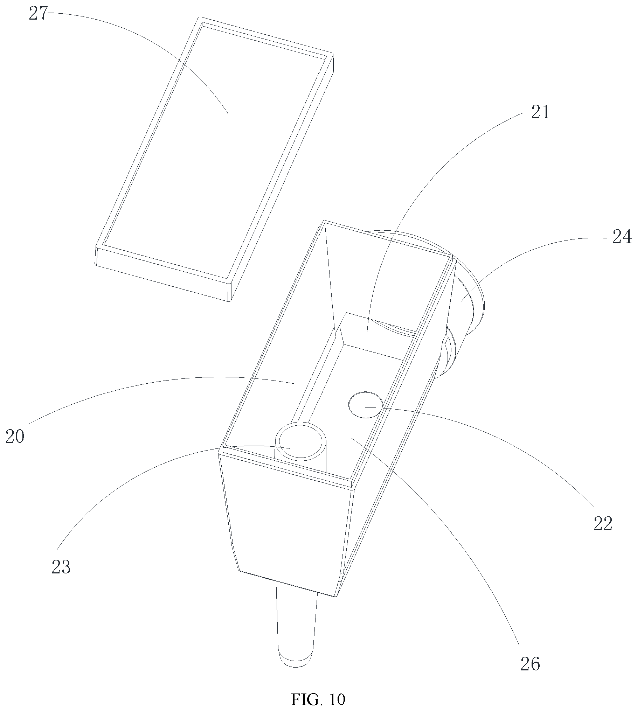

In addition, in the embodiments of the present application, as shown in FIG. 10, the liquid injection joint 22 and the liquid drainage joint 23 are disposed on the same side of an outer wall of the stent 2, wherein the stent 2 comprises a top plate 26 and a bottom plate 27 opposite to the top plate 27, and the liquid drainage joint 23 extends from an outside of the stent 2, passes through the top plate 26, extends into the liquid filtration cavity 20, and extends from one side where the liquid drainage joint 23 is located to another side of the liquid filtration cavity 20, that is, extends from the top plate 26 to the bottom plate 27. In this way, the liquid inside the liquid filtration cavity 20 can be drained completely to prevent a liquid residue. Meanwhile, the liquid drainage joint 23 further plays a limiting role; when the liquid injection hole 13 docks with the liquid injection joint 23, the first end of the body 1 abuts against the liquid drainage joint 23, thus playing a role in limiting an insertion depth of the filter 1 and preventing an excessive insertion action from damaging the apparatus.

Finally, it should be noted that the above embodiments are only used to illustrate the technical solutions of the present invention, but not to limit them. Under the thinking of the present invention, the above embodiments or technical features in different embodiments may also be combined, steps may be implemented in any order, and there are many other variations in different aspects of the present invention as described above. For the sake of simplicity, they are not provided in detail. Although the present invention is described in detail with reference to the aforementioned embodiments, those of ordinary skill in the art should understand that they may still modify the technical solutions recorded in the aforementioned embodiments or make equivalent substitutions for some of the technical features therein. However, these modifications or substitutions do not cause the essence of the corresponding technical solutions to depart from the scope of the technical solutions in the embodiments of the present invention.

## Claims

1. A filter applied to a polyp recovery apparatus, comprising:
a body, wherein the body comprises an upper shell and a lower shell that are buckled to each other, and an accommodating cavity for recovering polyps is formed between the upper shell and the lower shell;
a liquid injection hole formed in the upper shell and used for aspirated liquid to enter the accommodating cavity;
a liquid filtration mechanism disposed on the lower shell and used for filtering out moisture inside the accommodating cavity to filter the polyps.

2. The filter applied to a polyp recovery apparatus according to claim 1, wherein the body has a first end and a second end opposite to the first end, and the upper shell and the lower shell are hinged at the first end of the body.

3. The filter applied to a polyp recovery apparatus according to claim 2, wherein the first end of the body is provided with a first reset member for rotating the upper shell relative to the lower shell toward an open state.

4. The filter applied to a polyp recovery apparatus according to claim 3, wherein the first reset member is a torsion spring disposed at a hinge joint between the upper shell and the lower shell, the torsion spring is provided with a first support leg and a second support leg, the upper shell and the lower shell are respectively provided with an upper mounting portion and a lower mounting portion for assembling the torsion spring at the first end of the body, the upper mounting portion is disposed opposite to the lower mounting portion, the first support leg is disposed at the upper mounting portion and abuts against an inner surface of the upper shell, and the second support leg is disposed at the lower mounting portion and abuts against an inner surface of the lower shell.

5. The filter applied to a polyp recovery apparatus according to claim 3, wherein the polyp recovery apparatus comprises a stent for fixing the filter, the stent is provided with an assembly port used for allowing the filter to be inserted therein, and the second end of the body is provided with a sealing device for sealing the assembly port.

6. The filter applied to a polyp recovery apparatus according to claim 5, wherein the sealing device comprises a sealing plug fitted with the assembly port, and the sealing plug is fixed at the second end of the body.

7. The filter applied to a polyp recovery apparatus according to claim 5, wherein the second end of the body is provided with a guide column, the guide column extends along a direction away from the first end of the body, the sealing device comprises a piston slidable along the guide column, and the filter further comprises an adjustment device for adjusting relative positions of the piston and the guide column.

8. The filter applied to a polyp recovery apparatus according to claim 7, wherein the adjustment device comprises a handle disposed at one end of the guide column away from the body, the piston is located between the handle and the lower shell, and the handle is movably connected to the guide column for adjusting relative positions of the piston and the assembly port by moving the handle.

9. The filter applied to a polyp recovery apparatus according to claim 8, wherein the handle is slidingly connected to the guide column, and the handle is capable of controlling the piston to move along the guide column when sliding along the guide column.

10. The filter applied to a polyp recovery apparatus according to claim 8, wherein the handle is rotationally connected to the guide column, the piston abuts against the handle, the handle has a first station and a second station on a rotation track, and when the handle rotates from the first station to the second station, the handle pushes the piston toward a direction of the main body.

11. The filter applied to a polyp recovery apparatus according to claim 10, wherein the handle and the guide column are rotationally connected through a rotating shaft, one end of the handle opposite to the piston is provided with a first contact face and a second contact face, there is an included angle between the first contact face and the second contact face, a distance between the rotating shaft and the first contact face is smaller than that between the rotating shaft and the second contact face, the first contact face abuts against an end face of the piston when the handle is located at the first station, and the second contact face abuts against the end face of the piston when the handle is located at the second station.

12. The filter applied to a polyp recovery apparatus according to claim 11, wherein the second contact face matches the end face of the piston, and when the handle is located at the second station, a self-locking structure for preventing the handle from rotating is formed at a position of the rotating shaft opposite to the second contact face.

13. The filter applied to a polyp recovery apparatus according to claim 7, wherein one end of the guide column adjacent to the lower shell is further provided with a baffle for preventing the piston from curling.

14. The filter applied to a polyp recovery apparatus according to claim 13, wherein the sealing device further comprises a sealing ring, and a side face of the baffle is provided with a clamping slot for fixing the sealing ring.

15. The filter applied to a polyp recovery apparatus according to claim 13, wherein an end face of the baffle is provided with an air vent hole for being in communication with a liquid filtration cavity through the assembly port, and the piston covers the air hole when being fitted with the baffle.

16. The filter applied to a polyp recovery apparatus according to any one of claims 8-15, wherein the adjustment device further comprises a second reset member for providing a restoring force for the piston to slide toward a direction of the handle.

17. The filter applied to a polyp recovery apparatus according to claim 16, wherein the second reset member is a compression spring disposed between the piston and the lower shell.

18. A stent applied to a polyp recovery apparatus, comprising:
a liquid filtration cavity used for accommodating the filter according to any one of claims 1-17;
an assembly port disposed on a surface of the stent and being in communication with the liquid filtration cavity for allowing the filter to be inserted into the liquid filtration cavity;
a flow channel joint comprising a liquid injection joint and a liquid drainage joint, wherein the liquid injection joint is used for being in communication with the liquid injection hole of the filter, and the liquid drainage joint is in communication with the liquid filtration cavity for draining liquid inside the liquid filtration cavity.

19. The stent applied to a polyp recovery apparatus according to claim 18, wherein
the assembly port is fitted with a cross section of the body of the filter when buckled, so that when the filter is inserted into the assembly port, the upper shell and the lower shell are automatically buckled under an action of the assembly port.

20. The stent applied to a polyp recovery apparatus according to claim 18, further comprising a sealing interface fixed to the assembly port, wherein the second end of the filter is provided with a sealing device, and the sealing interface is fitted with the sealing device for achieving a sealed connection between the filter and the assembly port when the filter is inserted into the assembly port.

21. The stent applied to a polyp recovery apparatus according to claim 20, wherein a guide portion extending circumferentially in a funnel shape is provided on one side of the sealing interface away from the assembly port.

22. The stent applied to a polyp recovery apparatus according to claim 18, wherein a positioning protrusion is provided at a connection between the liquid injection joint and an inner wall of the stent, the positioning protrusion protrudes from the inner wall of the stent, and the positioning protrusion is fitted with the liquid injection hole of the filter, so that when the filter enters the liquid filtration cavity, the positioning protrusion slides into the liquid injection hole.

23. A polyp recovery apparatus, comprising:
the filter according to any one of claims 1-17;
the stent according to any one of claims 18-22.
